# EUROPEAN PATENT APPLICATION

(11) **EP 4 523 723 A1**
(43) Date of publication of application: **19.03.2025**
(21) Application number: 23198005.3
(22) Date of filing: 18.09.2023
(51) Int. Cl.: A61M 16/06

(54) **IMPROVED MINIMAL CONTACT RESPIRATORY NASAL MASK**

(71) Applicant: Air Liquide Medical Systems, 92182 Antony Cedex (FR)
(72) Inventor: ALBERICI, Luca, 25073 Bovezzo (IT); ROMAIOLI, Roberto, 25073 Bovezzo (IT); RUOCCO, Alessandra, 25073 Bovezzo (IT)
(74) Representative: Air Liquide

(57) **Abstract**

A respiratory nasal mask (1) comprising a mask body (10) comprising a front section (110) comprising a front opening (11) and a hollow flexible cushion (20) comprising a tubular front part (210) comprising a front aperture (21) and second fastening means (230), and a supple rear part (220) comprising peripheral edge regions (24) surrounding the rear breathing opening (22) that are configured to, in use, come into contact and provide a gas sealing with areas located immediately around the nostril edges of the user (P). The flexible cushion (20) further comprises a protruding element (250) arranged on the tubular front part (210) of said flexible cushion (20), and the mask body (10) further comprises a lodging (150) configured for receiving at least a part of the protruding element (250), when the flexible cushion (20) is attached to the mask body (10).

## Description

The invention concerns a respiratory nasal mask comprising a mask body and a flexible nasal cushion detachably coupled or fixed together, that is useable in the treatment of respiratory conditions or diseases affecting adults, children, toddlers, infants or newborns, such as obstructive sleep apnea (OSA).

Respiratory masks are commonly used for delivering a flow of breathable gas, such as air under pressure, for, or to assist in, patient respiration.

A mask assembly typically comprises a rigid or semi-rigid hollow shell or body, usually made of polymer, and further comprising a soft face-contacting cushion, commonly called a flexible cushion, that comes into contact with the patient's face and conforms to the various facial contours of the patient face for receiving the patient's nose and/or mouth so that the patient can inhale the gas comprised into a breathing chamber that is defined by the hollow shell or body and/or the flexible cushion. The flexible cushion is usually made of soft, resilient elastomeric material, such as soft silicone or similar material.

A gas supply line delivers a respiratory gas, such as air under pressure, to the mask, namely the breathing chamber. An example of such a mask is given by EP-A-462701.

Further, a headgear, comprising flexible straps or similar structures, is usually attached to the mask for correctly positioning, holding and/or securing the mask on the head of a patient. The headgear can be adjusted to pull the mask against the face with sufficient force to achieve a gas tight seal between the mask and the wearer's face as taught by EP-A-462701, EP-A-874667, EP-A-1972357 or WO-A-00/57942.

Other types of masks comprising a front shroud adapted to attach the headgear are disclosed by EP-A-2708258 or US-A-2007/0044804.

Masks receiving only the patient's nose are called a nasal mask, whereas those receiving the patient's nose and mouth are called facial or oro-nasal masks. Such masks are commonly used for treating respiratory conditions or diseases affecting adults or children, including some toddlers, infants or newborns, such as obstructive sleep apnea (OSA). For instance, the treatment of OSA or the like, for being efficient, requires that the patient uses the nasal mask overnight, namely when he/she sleeps.

Despite the fact that a lot of different architectures or masks have been proposed in the prior, especially for treating OSA or the like, problems or drawbacks still exist with existing nasal masks.

Thus, one important problem is related to the discomfort of use for the patient. Various causes of discomfort exist, such as gas leaks due to a wrong assembling of the mask components, a poor positioning of the mask of the patient's face, loose settings of the headgear, poor tightness of the cushion...

For trying to overcome those drawbacks, an improved respiratory nasal mask architecture has been proposed that is compact, simple to assemble/disassemble, light and comfortable to wear, and ensures efficient positioning and securing of the mask on the patient's face, good comfort of use for the patients and an efficient gas tightness, i.e. seal, preventing the escape of gas. Such an improved respiratory nasal mask, also called a "minimal contact mask" (MCM), comprises a mask body detachably coupled to a hollow flexible cushion that comprises a supple rear part comprising a rear breathing opening for allowing, in use, gas exchanges with the nostrils of a user, e.g. a patient. The supple rear part comprises peripheral edge regions surrounding the rear breathing opening that are configured to, in use, come into contact and provide a gas sealing with areas located immediately around the nostril edges of the user (P) comprising an upper lip area (ULA) located immediately under the nostrils, two curved alae (CA) on both sides of the nostrils and a tip region (TR) of the nose.

In a MCM, the cushion and the mask body should be easily detachable and re-attachable as a patient has to disassemble and re-assemble said components on regular basis, for instance every morning for cleaning them up or for replacement when the cushion is worn out.

In practice, it has been noticed that a lot of patients do not re-assemble correctly the cushion and the mask body, but often upside down, which generally leads to gas leaks and discomfort when the mask is subsequently used, i.e. donned by the patient.

In this context, a problem is to avoid such a wrong re-assembling or coupling of the main components of a MCM, namely the cushion and the mask body, when said components have been disassembled for cleaning operations or for replacement purposes.

A solution of the present invention concerns a respiratory nasal mask, namely a so-called "minimal contact mask" (MCM), comprising:
- a mask body comprising :
   . a front section comprising a front opening and
   . a tubular rear section comprising a first inner passage and a rear opening, said rear opening being in fluid communication with the front opening via the first inner passage, and further comprising first fastening means and
- a hollow flexible cushion comprising :
   . a tubular front part comprising a front aperture and second fastening means, and
   . a supple rear part comprising a rear breathing opening for allowing, in use, gas exchanges with nostrils of a user, e.g. a patient, said rear breathing opening being in fluid communication with said front aperture, said supple rear part comprising peripheral edge regions surrounding the rear breathing opening that are configured to, in use, come into contact and provide a gas sealing with areas located immediately around the nostril edges of the user (P) comprising an upper lip area (ULA) located immediately under the nostrils, two curved alae (CA) on both sides of the nostrils and a tip region (TR) of the nose,
and wherein the first fastening means of the tubular rear section of the mask body cooperates with the second fastening means of the tubular front part of the flexible cushion for detachably fixing the hollow flexible cushion directly to the mask body, when the tubular rear section of the mask body is inserted into the tubular front part of the flexible cushion.

Further, in the respiratory nasal mask of the invention :
- the flexible cushion further comprises a protruding element arranged on the tubular front part of the flexible cushion, said protruding element projecting away from said tubular front part in the direction of the mask body, and
- the mask body further comprises a lodging, said lodging being configured for receiving at least a part of the protruding element, when the flexible cushion is attached to the mask body.

The respiratory nasal mask according to the present invention can further comprise one or more of the following features.

### Mask body

The mask body of the nasal mask according to the present invention can further comprise one or more of the following additional features:
- the tubular rear section of the mask body is coaxially (axis XX) inserted into the tubular front part of the flexible cushion.
- the mask body comprises a peripheral border, i.e. a peripheral edge.
- the lodging is formed in the peripheral border of the mask body.
- the lodging comprises or forms (e.g. constitutes or is) a notch, a cut or similar arranged in the peripheral border of the mask body.
- the lodging of the mask body, i.e. notch, cut or similar, is dimensioned for receiving, i.e. for lodging, the protruding element of the cushion.
- the lodging of the mask body and the protruding element of the cushion have complementary shapes.
- the lodging of the mask body has or comprises a trapezoidal shape or the like.
- the lodging, i.e. notch, cut or similar, is arranged in an upper part of the mask body.
- the lodging, i.e. notch, cut or similar, is arranged in an upper portion of the peripheral border of the mask body.
- the lodging, i.e. notch, cut or similar, is arranged above the front opening.
- the lodging is formed by a notch, cut or similar, traversing the mask body, i.e. the wall of the mask body.
- the front section of the mask body comprises two lateral arms projecting laterally on each side of said mask body, i.e. on the left and right sides of the mask body, and in opposite directions.
- the two lateral arms are made one piece with the mask body.
- the two lateral arms and the mask body are made of polymer material, preferably a rigid or semi-rigid polymer material.
- the rigid or semi-rigid polymer material, i.e. a plastic material, is polycarbonate (PC), polypropylene (PP), Nylon^{®}, or the like, for instance PC.
- the two lateral arms have an elongated shape, such as a band or ribbon shape. Other shapes are of course also possible.
- the lateral arms have each a length of about 3 to 10 cm, preferably of about 3 to 6 cm.
- each lateral arm comprise a free end with headgear connecting structures for attaching a headgear thereto.
- the headgear connecting structures comprise one or several slots, holes, hooks or the like.
- the front section of the mask body comprises a front wall traversed by the front opening, preferably a curved front wall.
- the front section of the mask body comprises two opposite surfaces, namely an outer front surface (i.e. toward the outside) and a rear surface facing the cushion (i.e. toward the inside).
- the lodging traverses the front section of the mask body thereby joining its two opposite surfaces.
- the front wall constitutes a body structure having a length (L) of between 40 and 70 mm and a width (W) of between 30 and 50 mm.
- the front wall has a thickness of between 2 and 15 mm.
- the front wall is flat.
- the front wall is slightly foldable, i.e. can be slightly bent, especially when tension forces are applied by the headgear connected to the two arms.
- the front wall comprises two opposite tips, the two lateral arms being attached to said two opposite tips.
- the front opening is arranged at the center of the front wall, namely is a central opening.
- the front opening has a circular shape.
- the front opening has a diameter of between 15 and 25 mm, preferably of between 17 and 22 mm.
- the front section of the mask body, in particular the front wall, comprises a rear surface comprising, i.e. carrying, a collar structure (i.e. a tubular structure) projecting away from said rear surface and comprising a second inner passage (i.e. a lumen) in fluid communication with the front opening and with the first inner passage of the tubular rear section.
- the collar structure projects toward the tubular rear section and/or toward the cushion.
- the collar structure projects axially (XX).
- the tubular rear section has an oval contour, i.e. an oval outer perimeter. "Oval" means strictly oval or ellipsoidal.
- the tubular rear section and the collar structure are coaxially (XX) arranged.
- the tubular rear section and the collar structure are coaxially (XX) arranged and at a distance from each other, i.e. spaced by a spacing of several millimeters.
- the tubular rear section is attached to the rear surface of the front wall of the mask body, preferably formed in one piece, e.g. molded in one piece.
- the tubular rear section, the collar structure and the front wall of the mask body are formed in one piece, e.g. molded in one piece.
- the front section of the mask body further comprises several venting orifices, i.e. holes, slots or similar, traversing the front wall and arranged around the front opening.
- the venting orifices are in fluid communication with the inner breathing chamber of the flexible nasal cushion.
- the venting orifices are arranged in groups or arrays comprising each several venting orifices.
- groups or arrays of venting orifices are arranged so as to sandwich the front opening of the mask body, i.e. arranged to the left and to right of the front opening.
- the venting orifices traverse the front wall and open on the rear surface of the mask body between the collar structure and the tubular rear section of the mask body.
- the venting orifices open in the spacing between the tubular rear section and the collar structure of the mask body.
- the venting orifices are configured for venting to the atmosphere at least a part of the CO₂-contiaining gaseous flow expired by the user during the expiratory phases.
- the venting orifices have a cylindrical or tronconical shape, preferably their section is greater on the rear surface of the mask body than on the front surface of said mask body.
- the front opening and the collar structure of the mask body, and the front aperture of the flexible cushion are coaxially-arranged (XX).

### Cushion

The cushion of the nasal mask according to the present invention can further comprise one or more of the following additional features:
- the protruding element projecting away from said tubular front part in the direction parallel to the axis XX.
- the protruding element of the cushion has or comprises a trapezoidal shape or the like.
- the protruding element is arranged on an upper part of the cushion.
- the protruding element projects away from the peripheral edge or annular outer border of the tubular front part of the cushion.
- the protruding element is made one-piece with the tubular front part of the cushion.
- the protruding element is molded in one-piece with the tubular front part of the cushion.
- it comprises an inner breathing chamber, i.e. an inner room or chamber for the gases, i.e. gas delivered to the user, such as a patient, during inspiratory phases and/or CO₂-enriched gases exhaled by the user during expiratory phases.
- it is sized and designed for not covering the regions of the nose of the user situated above his/her nose tip or tip region (TR), when the mask is worn by said user, especially the dorsum, i.e. cartilaginous and/or bony dorsum.
- it is made of a flexible material, i.e. a supple and soft or semi-soft material.
- it is made of silicone or the like.
- the protruding element is also made of silicone or the like.
- the inner breathing chamber of the cushion comprises the rear opening.
- the rear opening is in fluid communication with the inner breathing chamber.
- the front aperture or front opening is in fluid communication with the inner breathing chamber of the cushion.
- the front aperture has an oval shape, i.e. an oval section. "Oval" means strictly oval or ellipsoidal.
- the front aperture of the cushion has an oval shape that matches the oval contour of the tubular rear section of the mask body.
- the dimensions of the oval shape of the front aperture of the cushion are approximatively equal to the dimensions of the oval contour of the tubular rear section of the mask body so that the tubular rear section can be tightly inserted and secured into the front aperture of the cushion.
- the front aperture of the cushion is delimited by an inner border that surrounds the tubular rear section of the mask body, when the tubular rear section of the mask body is inserted into the tubular front part of the flexible cushion.
- the rear opening of the cushion has (almost or quasi) rectangular or trapezoidal shape.
- the peripheral edge regions surrounding the rear opening of the flexible cushion comprise a peripheral thin wall, such as a membrane or the like, constituting a sealing, preferably a supple sealing skirt, around said rear opening so as to ensure an efficient gas tightness while in contact with the user's face, when the latter wears the mask, preferably all along the outer periphery of said rear opening.
- the peripheral thin wall of the peripheral edge regions of the cushion forming the sealing comes into contact and provide a gas sealing with the areas located immediately around the nostril edges of the use, namely the upper lip area (ULA), the two curved alae (CA), and the tip region (TR) of the nose.
- the rear surface of the flexible cushion is curved, in particular for matching, in use, the two curved alae (CA) of the nose of the user.
- the peripheral edge regions surrounding the rear opening of the flexible nasal cushion form a curved (quasi) contacting surface, preferably a triangular or trapezoidal curved contacting surface.
- the curved (quasi) contacting surface surrounding the rear opening comes into contact, in use, i.e. when the user wears the mask, with the areas (i.e. user's facial areas) located immediately around the nostril edges of the user thereby providing an efficient gas sealing.
- the rear opening having a (almost or quasi) rectangular or trapezoidal shape is arranged in the curved (quasi) triangular contacting surface located around the rear breathing opening, preferably in the center of said curved (quasi) triangular contacting surface.
- the peripheral edge regions of the rear opening of the flexible cushion comprises a base border, two lateral panels or wings, and a top border.
- the width of the top border is larger/greater than the width of the base border, for instance the width of the top border is of between 17 and 25 mm, whereas the width of the base border is of between 10 and 20 mm.
- the inner breathing chamber of the cushion is in fluid communication with the first inner passage of the tubular rear section of the mask body so that gas/gases can travel from the first inner passage of the tubular rear section of the mask body to the inner breathing chamber of the cushion, and vice versa, i.e. in both ways.
- the peripheral thin wall forming the sealing skirt around the rear breathing opening is molded in one piece with the rest of the cushion.
- a bridge element or support-element is arranged in the rear opening.
- the bridge element has an arch-shape or the like that projects into the cushion.
- the bridge element has a "U" or "V"-shape or similar.
- the bridge element forms a bridge between the upper and lower edges of the rear opening.
- when the mask is worn, the bridge element is in contact with the outer region of the nose of the patient separating the nostrils so as to constitute a support or the like prohibiting a collapse, sagging or similar of the thin regions of the cushion, namely the peripheral edge regions of the rear opening of the flexible cushion, in particular the base and top borders.
- the peripheral edge regions of the rear opening of the flexible cushion are thinner than the tubular front part regions of the flexible cushion.
- the thickness of the wall of the flexible cushion in the peripheral edge regions of the rear opening is less than 1mm, preferably of between 0.30 and 0.6 mm.
- the thickness of the wall of the flexible cushion in the tubular front part regions of the flexible cushion is comprised between 1 and 5 mm, preferably of at least 2 mm.
- the flexibility of the cushion is greater in the peripheral edge regions of the rear opening than in the tubular front part regions of said flexible cushion.
- the supple rear part of the cushion can have a first width W1 of between 45 and 70 mm, preferably of between 50 and 65 mm.
- the tubular front part of the cushion can have a second width W2 (with W2<W1) of between 35 and 55 mm, preferably of between 40 and 50 mm.
- the cushion can have a length L of between 25 and 50 mm, preferably of between 30 and 45 mm, more preferably of between 35 and 40 mm.
- the bridge element can have a third width W3 of between 1 and 6 mm, preferably of between 2 and 5 mm.
- the cushion can have a thickness T of between 25 and 45 mm, preferably of between 30 and 40 mm.

### Connecting structures

The mask body and the cushion of the nasal mask according to the present invention comprise connecting structures for attaching the cushion directly to the the mask body, in a detachable manner so that the user can disassemble them for cleaning operations for instance.

Attaching "directly" the cushion to the mask body means that no intermediary piece or element, such as an annular ring or the like, is used for attaching them together.

Said connecting structures comprise the first fastening means of the tubular rear section of the mask body that cooperate with the second fastening means of the tubular front part of the flexible cushion for detachably fixing the hollow flexible cushion directly to the mask body, without the need of any intermediary member, such as a ring or similar.

Those connecting structures comprise can further comprise one or more of the following additional features:
- the first fastening means of the mask body comprise an annular shoulder or flange projecting outwardly, preferably radially.
- the annular shoulder or flange is arranged on the tubular rear section of the mask body, preferably on the outer peripheral wall of the tubular rear section, i.e. around its periphery.
- the annular shoulder or flange is arranged in the region of the rear opening of the tubular rear section of the mask body, i.e. close to said rear opening.
- the second fastening means of the flexible cushion comprise an annular groove arranged in the inner peripheral wall of the tubular front part of the flexible cushion.
- the annular shoulder or flange of the mask body is lodged and retained into the annular groove of the flexible cushion, when the hollow flexible cushion is attached to the mask body.
- the annular groove of the flexible cushion is laterally delimited by a first side wall and a second side wall facing each other.
- the annular shoulder or flange is sandwiched between said first side wall and second side wall, when said annular shoulder or flange is lodged into the annular groove of the flexible cushion.
- the said first side wall and second side wall constitute abutments that limit or block the course or motion of the annular shoulder or flange of the mask body into the annular groove of the flexible cushion.
- the first fastening means of the mask body cooperate with the second fastening means of the flexible cushion for further ensuring a gas tightness, in particular between the annular shoulder or flange and the annular groove, when said annular shoulder or flange of the mask body is lodged and retained into the annular groove of the flexible cushion, thereby avoiding gas leaks or similar.

### Hollow gas connector and flexible hose

A flexible hose can be fluidly connected to the front opening of the mask body of the nasal mask according to the present invention, by means of a hollow connector arranged at a proximal free end of said flexible hose.

The hollow connector can comprise one or more of the following additional features:
- it is a straight connector, i.e. tubular shape, preferably having a cylindrical shape.
- it comprise an inner passage for the gas, i.e. a lumen.
- it is inserted into and held in position in the mask body, through the front opening of the front section of the mask body.
- it is detachably fixed to the mask body.
- it is rotatable with respect to the mask body.
- it is configured for connecting a flexible hose thereto, i.e. a gas line or the like. In other words, the tubular connector is adapted for receiving a flexible conduct or hose that feeds a respiratory gas under pressure to the respiratory mask. The gas is fed into the flexible gas conduct or hose by a gas source, such as a medical ventilator, a medical gas-delivery device or the like.
- it is in fluid communication with the breathing chamber of the cushion thereby allowing gas to circulate from the hollow connector to the cushion or in the opposite way.
- it is inserted into the collar structure through the front opening of the mask body and secured therein, i.e. firmly hold therein, for instance by a snap-fit connection, a press-fit connection or others.
- it comprises a proximal end that is secured into the collar structure of the front section of the mask body, in particular inserted through the front opening.
- the proximal end of the hollow connector has a cylindrical or tronconical shape.
- according to another embodiment, the connector maybe a hollow curved or elbow connector, i.e. having a "L-shape" or the like.
- it is made of polymer material, e.g. plastic.

### Headgear

The nasal mask according to the present invention can further comprise a headgear comprising one or more of the following additional features:
- it is attached to the headgear connecting structures of the two lateral arms.
- it comprises straps that are used for positioning, maintaining and/or securing the mask in a desired position on the facial regions of the patient.
- the straps are made of polymer material or fabric material, or both.
- the straps comprise or are elongated flexible bands, ribbons or the like.
- the straps constitute a tridimensional structure.
- the straps comprise Velcro^{®}-like portions for forming loops or the like and allowing attaching the headgear to the headgear connecting structures of the mask body.

### Respiratory assembly

The invention also concerns a respiratory assembly or installation for providing gas to a patient (P) comprising a gas delivery device, such as a medical ventilator, i.e. a respiratory assistance device or the like, and a nasal mask according to the present invention, wherein the gas delivery device is connected to the nasal mask by means of the flexible hose.

Such a respiratory assembly comprising a nasal mask according to the present invention is usable in the treatment of respiratory conditions or diseases affecting adults, children, toddlers, infants or newborns, such as obstructive sleep apnea (OSA).

A non-limitative embodiment of a respiratory nasal mask according to the present invention is shown in the enclosed Figures, among which:
- Figure 1 represents a side view of a patient equipped with a nasal mask according to one embodiment of the present invention,
- Figure 2 is a front view of Figure 1,
- Figure 3 is an enlarged view of a nasal mask according to the present invention, showing the cushion detached from the mask body.
- Figure 4 is lateral view of the cushion of a nasal mask according to the present invention.
- Figure 5 shows the dimensions of the cushion of a nasal mask according to the present invention.
- Figure 6 is sectional view of a nasal mask according to the present invention.

The present invention proposes a respiratory nasal mask 1, also called a minimal contact mask (MCM), as shown in Figures that illustrate an embodiment of a nasal mask 1 according to the present invention.

A nasal mask 1 according to the present invention comprises two main parts assembled or coupled together, namely a mask body 10 and a flexible cushion 20 attached together in a detachable manner so that they can be disassembled for instance for cleaning operations or for replacing a worn out cushion 20, and easily reassembled afterwards.

This mask 1 has a simple and light structure, and further facilitates the assembling/disassembling of the different components of the mask 1 by the users, namely the patients, especially during daily cleaning operations. Such a mask 1 further exhibits a good comfort as it is lighter and has a low risk of gas leaks.

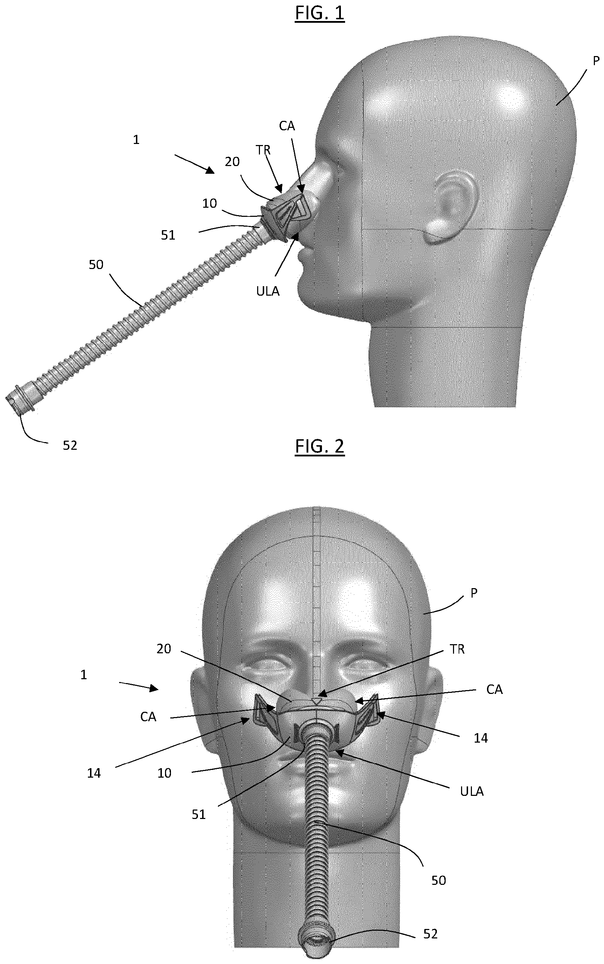

As shown in Fig. 1-2, a respiratory gas, such as air, is provided by a flexible hose 50 fluidly and mechanically connected to the mask body 10 by means of a tubular connector 51 arranged at the proximal end of said flexible hose 50. The distal end of the flexible hose 50 comprise an additional tubular connector 52 for fluidly and mechanically connecting the flexible hose 50 to a medical ventilator (not shown) or a similar apparatus that provides the respiratory gas to the flexible hose 50.

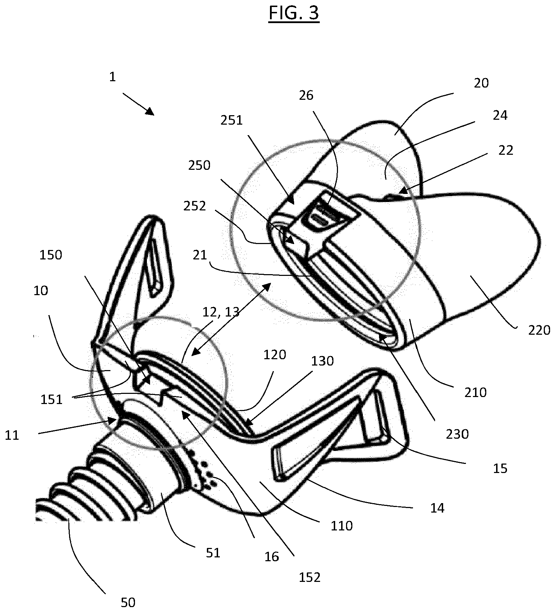

A nasal mask 1 of the present invention comprises a mask body 10 comprising two lateral arms 14 projecting laterally, in opposite directions, i.e. one toward the right side of the mask body 10 and the other toward the left side of the mask body 10 as shown in Figures 3 and 6-8 for instance. The two lateral arms 14 are arranged around a front opening 11 traversing the mask body 10.

The two lateral arms 14 that project laterally may also constitute right and left cheek supports, when the mask is worn by a patient, as shown in Fig. 1&2. Preferably, the two lateral arms 14 have elongated shapes, such as band or ribbon shapes, and/or can be slightly incurved to better match, if desired, some of the contours of the cushion 20. Each lateral arm 14 has for instance a length of about 3 to 10 cm, preferably of about 3 to 6 cm.

Further, the lateral arms 14 comprise a free end equipped with headgear connecting structures 15, such one or several slots, holes, hooks or the like. These headgear connecting structures 15 are designed for attaching a headgear thereto (not shown), typically the flexible straps of a headgear. A headgear is commonly used for maintaining and securing the mask 1 in a desired position on the head of the patient, as explained below. Preferably, the flexible straps of the headgear are made of polymer or fabric materials.

The mask body 10 of the nasal mask 1, as shown in the Figures, comprises a front section 110 comprising a front opening 11 and a tubular rear section 120 comprising a first inner passage 13, i.e. a lumen or the like, and a rear opening 12. The rear opening 12 is in fluid communication with the front opening 11 via the first inner passage 13

The tubular rear section 120 further comprises first fastening means 130 for attaching the mask body 10 to the cushion 20, as explained below.

Further, the nasal mask 1 comprises a hollow flexible cushion 20 comprising a tubular front part 210 comprising a front aperture 21 and second fastening means 230 cooperating with the first fastening means 130, and further comprising a supple rear part 220 comprising a rear breathing opening 22 for allowing, in use, gas exchanges with the two nostrils of a user P.

The hollow flexible cushion 20 further comprises an inner breathing chamber 23 for containing the gas, such as air, to be inhaled by the patient. The rear breathing opening 22 is in fluid communication with the front aperture 21, via the inner breathing chamber 23.

Further, the supple rear part 220 of the flexible cushion 20 comprises peripheral edge regions 24 surrounding the rear breathing opening 22 that are configured to, in use, come into contact and provide a gas sealing with areas located immediately around the nostril edges of the user P comprising an upper lip area (ULA) located immediately under the nostrils, two curved alae (CA) on both sides of the nostrils and a tip region (TR) of the nose, thereby providing a minimal contact surface or the like with facial areas of the user.

The tubular rear section 120 of the mask body 10 is coaxially (i.e. axis XX) inserted into the tubular front part 210 of the flexible cushion 20, when both are coupled together.

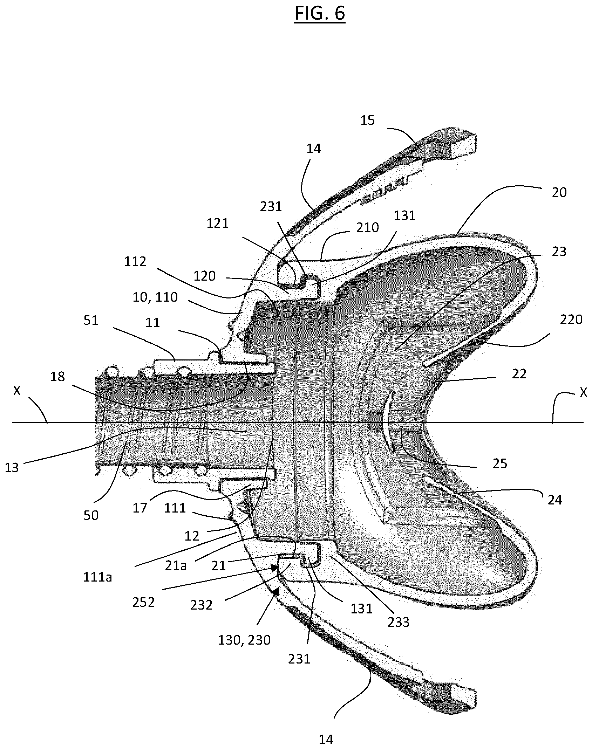

The first fastening means 130 of the tubular rear section 120 of the mask body 10 cooperate with the second fastening means 230 of the tubular front part 210 of the flexible cushion 20 for detachably fixing the hollow flexible cushion 20 directly to the mask body 10 as shown in Figure 6 (sectional view).

The mask body 10 comprises a front wall 111, preferably a curved and/or flat front wall 111, that is traversed by the front opening 11 that can have a circular shape and/or an inner diameter of about between 1 and 3 cm, for instance of between 15 and 25 mm. The front wall 111 of the front section 110 has a thickness of between 2 and 6 mm.

The mask body 10, in particular the front section 110 comprising the front opening 11 and the tubular rear section 120, and the two arms 14 are made of plastic material and molded in one piece, for instance by injection molding or any other suitable molding process. Typically, the plastic or polymer material can be PP, PC, PBT or nylon, or similar materials.

The front section 110 of the mask body 10 comprises two opposite surfaces 111a, 112, namely an outer front surface 111a (i.e. situated toward the outside) and a rear surface 112 facing the cushion 20 (i.e. situated toward the inside). The rear surface 112 of the mask body 10 comprises a collar structure 17, i.e. a tubular structure, projecting away from said rear surface 112. The collar structure 17 is axially (XX) traversed by a second inner passage 18, i.e. a lumen, in fluid communication with the front opening 11 and with the first inner passage of the tubular rear section 120 of the mask body 10. The collar structure 17 projects axially (XX) toward the tubular rear section 120 and toward the cushion 20. Preferably, the collar structure 17 is cylindrical.

The tubular rear section 120 and the collar structure 17 are coaxially (XX) arranged and located at a distance from each other, i.e. spaced by a spacing of several millimeters. The tubular rear section 120, the front section 110 and the collar structure 17 are preferably molded in one piece.

Further, the tubular rear section 120 has an oval contour 121, i.e. an oval outer perimeter. "Oval" means strictly oval or ellipsoidal.

Furthermore, as shown in Figure 3, the front section 110 of the mask body 10 further comprises several venting orifices 16, i.e. traversing holes, slots or similar, traversing the front wall 110 and arranged around the front opening 11. They are configured for venting to the atmosphere at least a part of the CO₂-contiaining gaseous flow expired by the user P during his/her expiratory phases.

The venting orifices 16 are preferably arranged in groups or arrays comprising each several venting orifices 16 each. For instance, in 2 arrays that sandwich the front opening 11 of the mask body 10, i.e. arranged to the left and to right of the front opening 11.

The venting orifices 16 are in fluid communication with the inner breathing chamber 23 of the flexible nasal cushion 20, via the tubular rear section 120. Preferably, the venting orifices 16 traverse the front wall 111 and open on the rear surface 112 of the mask body 10 between the collar structure 17 and the tubular rear section 120 of the mask body 10 as visible in the Figures, namely in the spacing between the tubular rear section 120 and the collar structure 17 of the mask body 10.

The venting orifices 16 can have a cylindrical or tronconical shape, preferably their section is greater on the rear surface 112 than on the front surface 110 of said mask body, i.e. they have preferably a tronconical shape.

The front opening 11, the tubular rear section 120 and the collar structure 17 of the mask body 10, and the front aperture 21 of the flexible cushion 20 are coaxially-arranged (XX).

Further, the hollow flexible cushion 20 constitutes a tridimensional hollow flexible structure defining the inner breathing chamber 23. It further comprises a front aperture 21 and a rear opening 22 that fluidly communicate with the inner breathing chamber 23. The cushion is preferably entirely made of silicone or the like.

When the mask 1 is worn, the supple rear part 220 of the flexible cushion 20 comprises a contact zone located on its outer surface that comes into contact, in use, with the patient's face, namely regions located immediately around the nostrils of the patient P, in particular the upper lip area (ULA) located immediately under the nostrils, two curved alae (CA) on both sides of the nostrils and the tip region (TR) of the nose as shown in Fig. 1&2.

Said contact zone comprises peripheral edge regions 24 surrounding the rear breathing opening 22 supple rear part 220, i.e. regions located immediately around the rear breathing opening 22. Said peripheral edge regions 24 are configured to, in use, come into contact and provide a gas sealing surface with areas located immediately around the nostril edges of the user P, i.e. the upper lip area (ULA), the two curved alae (CA) and the region (TR) of the nose of the user P.

The cushion 20 of the nasal mask 1 of the present invention has a compact size and does not extend over the top of the nose, namely does not cover the dorsum regions, i.e. cartilaginous and/or bony dorsum, when the mask 1 is worn by a user P as shown in Fig. 1&2, which reduces and/or minimizes the contact with the skin of the user as it rests only on the areas located immediately around the entrances of the nostrils of said user. In other words, the compact cushion 20 of the invention can be sized and/or designed in taking into account only the width of the bottom of the nose of the user.

Furthermore, in order to provide efficient gas tightness (i.e. seal) and/or good comfort for the patient, the cushion wall in the peripheral edge regions 24 surrounding the rear opening 22 of the supple rear part 220 of the cushion 20 is preferably designed or conceived as a thin membrane, i.e. a soft flexible membrane, that comes into contact with the patient's facial areas or regions, namely the upper lip area (ULA), the two curved alae (CA) and tip region (TR) as above stated, so as to better match his/her facial morphology. Said flexible thin membrane forms a soft skirt or the like all along the edges of the rear opening 22 of the cushion 20. A unique membrane is preferably used; however, in alternative embodiments, several membranes may be superimposed.

Actually, the rear outer surface of the cushion 30 that comprises the peripheral edge regions 24 surrounding the rear breathing opening 22, has a curved shape for better matching some nasal regions, in particular the two curved alae (CA) situated on both sides of the nostrils of the user P. It comprises a base or bottom border that comes, in use, into contact with the upper lip area (ULA) of the user, two lateral panels or wings that come, in use, into contact with the two curved alae (CA) on both sides of the nostrils of the user P, and a top border that comes, in use, into contact with the tip region (TR) of the nose of the user P.

The first width of the top border is greater than the second width of the base border. For instance, the first width is comprised between 17 and 25 mm, whereas the second width is comprised between 10 and 20 mm.

In other words, the cushion 30 has a tridimensional form or shape that well-matches the contours of the patient's nasal regions (i.e. ULA, CA, TR), in particular thanks to its curved rear surface, thereby forming a contact zone constituting a gas seal, i.e. improved tightness.

Further, the rear breathing opening 22 has a (quasi-) rectangular or similar (e.g. trapezoidal shape). It is arranged at the center of the curved rear surface including the peripheral edge regions 24 surrounding the rear breathing opening 23 of the supple rear part 220 of the flexible nasal cushion 20.

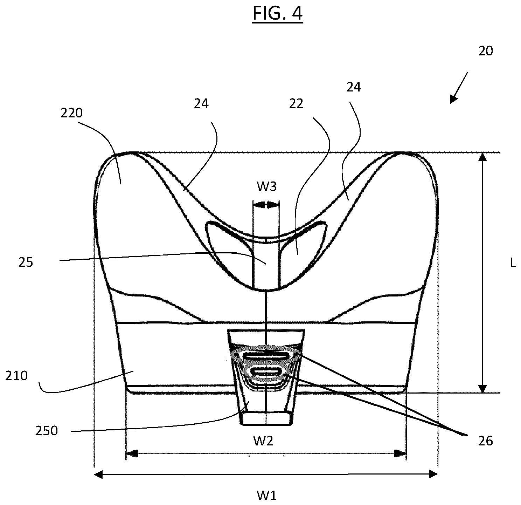

As shown in Figure 4, which is a lateral view of an embodiment of a nasal cushion 20 according to the present invention, the hollow flexible cushion 20 can further comprise a bridge element 25, such as a little band or the like, dividing the rear breathing opening 22, namely forming a bridge between the upper and lower edges of the rear breathing opening 22, i.e. in the supple rear part 220. The bridge element 25 can have an arch-shape, for instance a "U" or "V"-shape, that slightly projects into the inner breathing chamber 23 of the cushion 20.

When the nasal mask 1 is worn by the patient P, as shown in Fig. 1 & 2, the bridge element 25 comes into contact with the outer region of the nose of the patient separating the nostrils, namely the nasal columella, so as to constitute a support or the like prohibiting a collapse, sagging or similar of the thin regions of the supple rear part 220 of the cushion 20, namely the peripheral edge regions 24, in particular the base and top borders of the rear breathing opening 22.

The bridge element 25 is preferably molded in one-piece with the rest of the flexible cushion 20, in particular with the supple rear part 220 of the cushion 20. Furthermore, the tubular front part 210 of the cushion 20 comprises a front aperture 21, i.e. a gas opening, having an oval shape, i.e. an oval section. "Oval" means strictly oval or ellipsoidal. The oval shape of the front aperture 21 of the cushion 20 matches the oval contour of the tubular rear section 120 of the mask body 10, i.e. both have complementary oval forms. In other words, the dimensions of the oval shape of the front aperture 21 of the cushion 20 are approximately equal to the dimensions of the oval contour of the tubular rear section 120 of the mask body 10 so that the tubular rear section 120 can be tightly inserted and secured into the front aperture 21 of the cushion 20 as shown in Figure 6.

Further, the front aperture 21 of the cushion 20 is delimited by an inner border 21a that surrounds the tubular rear section 120 of the mask body 10, when said tubular rear section 120 of the mask body 10 is inserted into the tubular front part 210 of the flexible cushion 20.

According to the present invention, for ensuring an efficient coupling of the cushion 20 directly to the mask body 10, the tubular front part 210 of the flexible cushion 20 further comprises second fastening means 230 that are configured for cooperating with the first fastening means 130 of the tubular rear section 120 of the mask body 10.

In other words, for ensuring a firm and tight attachment or, conversely, for allowing a detachment of the cushion 20 to the mask body 10 for cleaning operations or the like, it is provided suitable connecting means or structures cooperating together, namely the first and second fastening means 130, 230. The first fastening means 130 arranged on the tubular rear section 120 of the mask body 10 comprise an annular shoulder or flange 131 projecting outwardly, preferably radially. Said annular shoulder or flange 131 is arranged on the outer peripheral wall of the tubular rear section 130, i.e. around its periphery, having an oval contour as above explained. The annular shoulder or flange is located in the region of the rear opening 12 of the mask body 10, i.e. close to said rear opening 12.

Further, the second fastening means 230 comprise an annular groove 231 arranged in the inner peripheral wall 211 of the tubular front part 210 of the flexible cushion 20 as shown in Figure 6. Said annular groove 231 is laterally delimited by a first side wall 232 and a second side wall 233 facing each other. When the hollow flexible cushion 20 is attached to the mask body 10, the annular shoulder or flange 131 of the mask body 10 is lodged and retained into the annular groove 231 of the flexible cushion 20, i.e. the annular shoulder or flange 131 is sandwiched between the first and second side walls 232, 233. The first and second side walls 232, 233 are linked, i.e. joined, by a bottom wall forming the bottom of the groove 231.

Actually, said first and second side walls 232, 233 constitute abutments or the like that limit or block the course or motion of the annular shoulder or flange 131 into the annular groove 231 of the flexible cushion 20.

As already explained, the cushion 20 and the mask body 10 have to be regularly disassembled for cleaning operations or for replacement purposes, and re-assembled afterwards. For minimizing or avoiding the risk that those components are not correctly re-assembled, i.e. wrongly coupled, for instance upside down, an "anti-error" coupling system has been arranged on the cushion 20 and on the mask body 10.

According to the invention, the "anti-error" coupling system comprises a protruding element 250 cooperating with a lodging 150 thereby forming a male/female assembly (cf. encircled areas in Fig. 3).

More precisely, the protruding element 250 is arranged on the tubular front part 210 of the flexible cushion 20, whereas the lodging 150 is arranged on the mask body 10, the protruding element 250 projecting away from the tubular front part 210 of the flexible cushion 20 and in the direction of the mask body 10, preferably parallel to the axis XX.

The lodging 150 is configured and/or dimensioned for receiving at least a part of the protruding element 250, when the flexible cushion 20 is attached to the mask body 10. Preferably, the protruding element 250 and the lodging 150 have complementary shapes, such as trapezoidal shapes as shown in Figure 3.

The lodging 150 is advantageously arranged in the peripheral border or edge of the mask body 10. The lodging 150 can be or comprise a notch, a cut or similar arranged in the peripheral border 151 of the mask body 10, in particular in an upper part or region 152 of the mask body 10, namely situated above the front opening 11, as shown in Fig. 3. The lodging 150 traverses the mask body 10, i.e. the front wall of the mask body 10, i.e. its front section, thereby joining its two opposite surfaces 111a, 112.

Further, the protruding element 250 arranged on the cushion 20 projects away from the tubular front part 210 of the cushion 20, in a direction parallel to the axis XX and toward the mask body 10.

The protruding element 250 has or comprises a trapezoidal shape or the like that matches the shape of the lodging 150, i.e. notch, cut or the like.

Preferably, the protruding element 250 is arranged on an upper part 251 of the tubular front part 210 of the cushion 20, while projecting away from the peripheral edge or annular outer border 252 of the tubular front part 210.

The protruding element can be advantageously made one-piece with the tubular front part 210 of the cushion 20, for instance by molding or similar.

For instance, the protruding element 250 has a length of about 2 mm to 20 mm, preferably of about 5 mm to 20 mm, whereas the lodging 150 has a width of about 2 mm to 20 mm, preferably of about 5 mm to 20 mm.

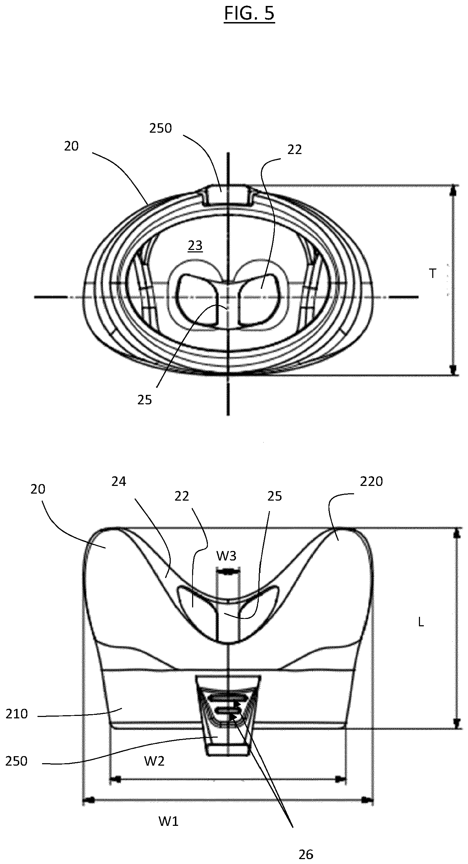

Figure 5 (and Fig. 4) shows the dimensions of the cushion 20 of a nasal mask 1 according to the present invention.

Those dimensions depends on the size of the cushion 20 that can exist in several sizes, such as small (S), medium (M) and/or large (L) sizes, so that it can fit various morphologies of patients.

For instance :
- the supple rear part 220 of the cushion 20 can have a first width W1 of between 45 and 70 mm, preferably of between 50 and 65 mm,
- the tubular front part 210 of the cushion 20 can have a second width W2 (with W2<W1) of between 35 and 55 mm, preferably of between 40 and 50 mm.
- the cushion 20 can have a length L of between 25 and 50 mm, preferably of between 30 and 45 mm, more preferably of between 35 and 40 mm.
- the bridge element 25 can have a third width W3 of between 1 and 6 mm, preferably of between 2 and 5 mm.
- the cushion 20 can have a thickness T of between 25 and 45 mm, preferably of between 30 and 40 mm.

Examples of dimensions of cushions of different sizes (S, M, L) are given in the below Table.

| Sizes | S | M-L |
|---|---|---|
| W1 | 53.5 mm | 62.4 mm |
| W2 | 43.6 mm | 43.6 mm |
| W3 | 4 mm | 4 mm |
| L | 37.4 mm | 39.6 mm |
| T | 35.4 mm | 35.4 mm |

Further, according to an embodiment, the cushion 20 can further comprise one or several relief structures 26 arranged on the upper part 251 of the cushion 20 in the region of the protruding element 250, for instance two little walls in relief, that help visually-impaired users to easily locate the protruding element 250. The relief structures 26 can be arranged on the protruding element 250 itself. Generally speaking, the compact nasal mask 1 of the present invention is light and comfortable to wear. Such a simple architecture leads to a reduction of the overall size and weight of the mask 1, allowing easy assembling and/or disassembling operations and efficient positioning and securing of the mask 1 on the patient's nasal regions, as well as a good tightness (i.e. seal) and an improved comfort of use for the patient.

The nasal mask 1 of the present invention can be used in a method for treatment of a respiratory disorder or condition affecting infant, toddler, child or adult patients, such as OSA or other respiratory troubles.

## Claims

1. Respiratory nasal mask (1) comprising:
- a mask body (10) comprising :
. a front section (110) comprising a front opening (11) and
. a tubular rear section (120) comprising a first inner passage (13) and a rear opening (12), said rear opening (12) being in fluid communication with the front opening (11) via the first inner passage (13), and further comprising first fastening means (130) and
- a hollow flexible cushion (20) comprising :
. a tubular front part (210) comprising a front aperture (21) and second fastening means (230), and
. a supple rear part (220) comprising a rear breathing opening (22) for allowing, in use, gas exchanges with nostrils of a user (P), said rear breathing opening (22) being in fluid communication with said front aperture (21), said supple rear part (220) comprising peripheral edge regions (24) surrounding the rear breathing opening (22) that are configured to, in use, come into contact and provide a gas sealing with areas located immediately around the nostril edges of the user (P) comprising an upper lip area (ULA) located immediately under the nostrils, two curved alae (CA) on both sides of the nostrils and a tip region (TR) of the nose, and wherein the first fastening means (130) of the tubular rear section (120) of the mask body (10) cooperates with the second fastening means (230) of the tubular front part (210) of the flexible cushion (20) for detachably fixing the hollow flexible cushion (20) directly to the mask body (10), when the tubular rear section (120) of the mask body (10) is inserted into the tubular front part (210) of the flexible cushion (20),
**characterized in that**:
- the flexible cushion (20) further comprises a protruding element (250) arranged on the tubular front part (210) of said flexible cushion (20), said protruding element (250) projecting away from said tubular front part (210) in the direction of the mask body (10), and
- the mask body (10) further comprises a lodging (150), said lodging (150) being configured for receiving at least a part of the protruding element (250), when the flexible cushion (20) is attached to the mask body (10).

2. Mask according to Claim 1, **characterized in that** the mask body (10) comprises a peripheral border (151) and the lodging (150) is formed in said peripheral border (151) of the mask body (10).

3. Mask according to any one of the preceding Claims, **characterized in that** the lodging (150) comprises or forms a notch or a cut arranged in the peripheral border (151) of the mask body (10).

4. Mask according to any one of the preceding Claims, **characterized in that** the lodging (150) is arranged in an upper portion (152) of the peripheral border (151) of the mask body (10).

5. Mask according to any one of the preceding Claims, **characterized in that** the lodging (150) is arranged above the front opening (11) of the mask body (10).

6. Mask according to Claim 1, **characterized in that** the protruding element (250) is arranged on an upper part (251) of the cushion (20).

7. Mask according to any one of Claims 1 and 6, **characterized in that** the lodging (150) of the mask body (10) and the protruding element (250) of the cushion (20) have complementary shapes.

8. Mask according to any one of Claims 6 and 7, **characterized in that** the protruding element (250) of the cushion (20) has or comprises a trapezoidal shape.

9. Mask according to any one of Claims 1 or 6 to 8, **characterized in that** the protruding element (250) projects away from the annular outer border (252) of the tubular front part (210) of the cushion (20).

10. Mask according to any one of Claims 1 or 6 to 9, **characterized in that** the protruding element (250) is made one-piece with the tubular front part (210) of the cushion (20).

11. Mask according to Claim 1, **characterized in that** the front section (110) of the mask body (10) further comprises two lateral arms (14) projecting laterally on each side of said mask body (10), preferably the two lateral arms (14) are made one piece with the mask body (10).

12. Mask according to Claim 1, **characterized in that** the front section (110) of the mask body (10) is traversed by several venting orifices (16) arranged around the front opening (11).

13. Mask according to Claim 1, **characterized in that** the cushion (20) is made of silicone.

14. Mask according to Claim 1, **characterized in that** the mask body (10) is made of polymer material.

15. Installation for providing gas to a patient (P) comprising a medical ventilator and a respiratory nasal mask (1) according to any one of the preceding Claims, said medical ventilator being fluidly and mechanically connected to the respiratory nasal mask (1) by means of a flexible hose (50) comprising a tubular hollow connector (51).

## Amended claims

### Amended claims in accordance with Rule 137(2) EPC.

1. Respiratory nasal mask (1) comprising:
- a mask body (10) comprising :
. a front section (110) comprising a front opening (11) and
. a tubular rear section (120) comprising a first inner passage (13) and a rear opening (12), said rear opening (12) being in fluid communication with the front opening (11) via the first inner passage (13), and further comprising first fastening means (130) and
- a hollow flexible cushion (20) comprising :
. a tubular front part (210) comprising a front aperture (21) and second fastening means (230), and
. a supple rear part (220) comprising a rear breathing opening (22) for allowing, in use, gas exchanges with nostrils of a user (P), said rear breathing opening (22) being in fluid communication with said front aperture (21),
and wherein the first fastening means (130) of the tubular rear section (120) of the mask body (10) cooperates with the second fastening means (230) of the tubular front part (210) of the flexible cushion (20) for detachably fixing the hollow flexible cushion (20) directly to the mask body (10), when the tubular rear section (120) of the mask body (10) is inserted into the tubular front part (210) of the flexible cushion (20),
wherein :
- the flexible cushion (20) further comprises a protruding element (250) arranged on the tubular front part (210) of said flexible cushion (20), said protruding element (250) projecting away from said tubular front part (210) in the direction of the mask body (10), and
- the mask body (10) further comprises a lodging (150), said lodging (150) being configured for receiving at least a part of the protruding element (250), when the flexible cushion (20) is attached to the mask body (10),
**characterized in that** :
- the supple rear part (220) comprises peripheral edge regions (24) surrounding the rear breathing opening (22) that are configured to, in use, come into contact and provide a gas sealing with areas located immediately around the nostril edges of the user (P) comprising an upper lip area (ULA) located immediately under the nostrils, two curved alae (CA) on both sides of the nostrils and a tip region (TR) of the nose,
- the peripheral edge regions (24) surrounding the rear opening (22) of the flexible cushion (20) form a curved contacting surface, said curved contacting surface coming into contact, in use, with the areas located immediately around the nostril edges of the user thereby providing an efficient gas sealing,
- the thickness of the wall of the flexible cushion (20) in the peripheral edge regions (24) of the rear opening (22) is less than 1mm, said peripheral edge regions (24) comprising a base border, two lateral wings, and a top border,
- a bridge element (25) is arranged in the rear opening, said bridge element (25) forming a bridge between upper and lower edges of the rear opening (22), and
- said bridge element (25) has an arch-shape that projects into the cushion (25), so that, when the mask (1) is worn, the bridge element (25) is in contact with the outer region of the nose of the patient separating the nostrils thereby prohibiting a collapse or sagging of the thin regions of the cushion (24) comprising the base and top borders.

2. Mask according to Claim 1, **characterized in that** the mask body (10) comprises a peripheral border (151) and the lodging (150) is formed in said peripheral border (151) of the mask body (10).

3. Mask according to any one of the preceding Claims, **characterized in that** the lodging (150) comprises or forms a notch or a cut arranged in the peripheral border (151) of the mask body (10).

4. Mask according to any one of the preceding Claims, **characterized in that** the lodging (150) is arranged in an upper portion (152) of the peripheral border (151) of the mask body (10) and the protruding element (250) is arranged on an upper part (251) of the cushion (20).

5. Mask according to any one of the preceding Claims, **characterized in that** the lodging (150) is arranged above the front opening (11) of the mask body (10).

6. Mask according to Claim 1, **characterized in that** the bridge element (25) has a "U" or "V"-shape or similar.

7. Mask according to any one of Claims 1 and 4, **characterized in that** the lodging (150) of the mask body (10) and the protruding element (250) of the cushion (20) have complementary shapes.

8. Mask according to any one of Claims 4 and 7, **characterized in that** the protruding element (250) of the cushion (20) has or comprises a trapezoidal shape.

9. Mask according to any one of Claims 1, 4, 7 or 8, **characterized in that** the protruding element (250) projects away from the annular outer border (252) of the tubular front part (210) of the cushion (20).

10. Mask according to any one of Claims 1, 4 or 7 to 9, **characterized in that** the protruding element (250) is made one-piece with the tubular front part (210) of the cushion (20).

11. Mask according to Claim 1, **characterized in that** the front section (110) of the mask body (10) further comprises two lateral arms (14) projecting laterally on each side of said mask body (10), preferably the two lateral arms (14) are made one piece with the mask body (10) or the front section (110) of the mask body (10) is traversed by several venting orifices (16) arranged around the front opening (11).

12. Mask according to Claim 1, **characterized in that**:
- the thickness of the wall of the flexible cushion (20) in the peripheral edge regions (24) of the rear opening (22) is of between 0.30 and 0.6 mm,
- the width of the top border is of between 17 and 25 mm, and
- the width of the base border is of between 10 and 20 mm.

13. Mask according to Claim 1, **characterized in that** the cushion (20) is made of silicone or the mask body (10) is made of polymer material.

14. Mask according to Claim 1, **characterized in that** the cushion (20) can further comprise one or several relief structures (26) arranged on the upper part (251) of the cushion (20) in the region of the protruding element (250), preferably the relief structures 26 are arranged on the protruding element (250).

15. Installation for providing gas to a patient (P) comprising a medical ventilator and a respiratory nasal mask (1) according to any one of the preceding Claims, said medical ventilator being fluidly and mechanically connected to the respiratory nasal mask (1) by means of a flexible hose (50) comprising a tubular hollow connector (51).
